# EUROPEAN PATENT APPLICATION

(11) **EP 0 793 966 A1**
(43) Date of publication of application: **10.09.1997**
(21) Application number: 97103522.5
(22) Date of filing: 04.03.1997
(51) Int. Cl.: A61K 38/13, A61K 9/06, A61K 9/10, A61K 9/107, A61K 47/26

(54) **Cyclosporin-containing topical pharmaceutical composition**

(30) Priority: 05.03.1996 KR 9605731
(71) Applicant: Hanmi Pharmaceutical Co., Ltd., Kyungki-do (KR)
(72) Inventor: Woo, Jong Soo, Jangan-gu, Suwon-shi, Kyungki-do, (KR); Song, Young Heon, Pyungtaek-shi, Kyungki-do (KR); Lee, Young Sin, Kyungki-do (KR); Shim, Chang Koo, Seoul (KR); Han Yong Hae, Seoul (KR); Lee, Young Joo, Pusan (KR)
(74) Representative: Dost, Wolfgang, Dr.rer.nat., Dipl.-Chem.

(57) **Abstract**

The present invention relates to a topical pharmaceutical composition containing cyclosporin as an active ingredient. More specifically, the present invention relates to an emulsion-containing hydrogel preparation prepared by a process which comprises mixing a self-emulsifying emulsion preconcentrate (A) consisting of cyclosporin as an active ingredient, a solubilizing and absorption-enhancing agent selected from amphiphilic non-alcoholic solvents having high boiling point of 200°C or more, a surfactant and an oil, and a hydrogel (B) consisting of a gelling agent and purified water, and adjusting the pH value of the mixture with an alkalizing agent. The topical preparation according to the present invention has advantages in that stability and skin permeability are good, the feeling when the preparation is applied to the skin is satisfactory, cyclosporin is very uniformly dispersed in the preparation to provide a good pharmacological activity, and it can be prepared in a simple and convenient manner.

## Description

### Background of the Invention

### 1. Field of the invention

The present invention relates to a topical pharmaceutical composition containing cyclosporin as an active ingredient. More specifically, the present invention relates to an emulsion-containing hydrogel preparation prepared by a process which comprises mixing a self-emulsifying emulsion preconcentrate (A) consisting of cyclosporin as an active ingredient, a solubilizing and absorption-enhancing agent selected from amphiphilic non-alcoholic solvents having high boiling point of 200°C or more, a surfactant and an oil, and a hydrogel (B) consisting of a gelling agent and purified water, and adjusting the pH value of the mixture with an alkalizing agent.

### 2. Background art

Cyclosporin is a specific macromolecular (molecular weight 1202.64) cyclic peptide compound consisting of 11 amino acids, which has useful pharmacological activities, particularly immunosuppressive activity and anti-inflammatory activity. Therefore, cyclosporin has been known as an immunosuppressant and widely used for suppression of rejections of the living body which are caused by transplantation of organs such as kidney.

Since 1984 it has been disclosed that cyclosporin has useful pharmacological activity for various diseases mainly caused by autoimmune response as well as the activity in the field of organ transplantation [see, Christopher E.M. Griffiths: Systemic and local administration of cyclosporin in the treatment of psoriasis, J. Am. Acad. Dermatol., 23, (6 part 2) 1242-1247 (1990)].

Specific examples of the autoimmune diseases to which cyclosporin can be applied include arthritis, autoimmune hematologic disorder, chronic bronchial asthma, systemic lupus erythematosus, polymyositis, systemic scleroderma, Wegener's syndrome, myasthenia gravis, psoriasis vulgaris, pustular psoriasis, autoimmune intestinal diseases (spontaneous ulcerative colitis, Crohn's disease), sarcoidosis, multiple sclerosis, juvenile diabetes, uveitis, psoriatic arthritis, glomerular nephritis, alopecia areata, contact dermatitis, atopic dermatitis, etc.

Particularly, cyclosporin is one of drugs which can be applied to patients suffering from severe psoriasis which can be hardly treated with conventional therapeutic agents. In 1979, during treatment of arthritis (psoriatic arthritis) Mueller, Hermann, etc. have first reported that cyclosporin has a useful effect on dermatologic disorders, and since then numerous similar reports have been continuously published. According to them, it has been identified that cyclosporin is very effective for treatment of psoriasis. In addition, Zachariae et. al. have reported that cyclosporin shows a siginificant effect on pustular psoriasis as well as psoriasis vulgaris, and further it has been disclosed that cyclosporin is also effective for treatment of palmoplantar pustulosis [see, Zachariae H. and Olsen T. S.: Efficacy of cyclosporin A in psoriasis; An overview of dose/response, indications, contraindications and side-effects. Clinical Nephrology, 43(3), 154-158 (1995), Laburte C., Grossman R. et al.: Efficacy and safety of oral cyclospoin A (CyA ; Sandimmun®) for long-term treatment of chronic severe plaque psoriasis. Br. J. Dermatol., 130, 366-375 (1994)].

According to the conventional method for treatment of psoriasis using cyclosporin, cyclosproin is administered per oral either in an amount of 6mg/kg/day for 2 months or in an amount of 14mg/kg/day for 2 weeks, as a short-term therapy. Alternatively, as a long-term therapy cyclosporin is administered in an amount of 1-4mg/kg/day for 12 to 25 months [see, Christopher E. M. Griffiths: Systemic and local administration of cyclosporin in the treatment of psoriasis, J. Am. Acad. Dermatol., 23, (6 part 2) 1242-1247 (1990)].

However, when cyclosporin is administered in such a high therapeutic amount for treament of psoriasis, it may cause severe side effects such as hypertension or renal toxicity [see, Fry L.: Cyclosporin A in the treatment of psoriasis; A review. J. Dermatol. Treat., 5 (suppl. 1) 52-54 (1994), Zachariae H. and Olsen T. S.: Efficacy of cyclosporin A in psoriasis; An overview of dose/response, indications, contraindications and side-effects. Clinical Nephrology, 43(3), 154-158 (1995)]. In addition, it has been disclosed that even though cyclosporin is administered in a high dose of 14mg/kg/day, the concentration of cyclosporin distributed in the skin is merely 3.5ng/mg. Therefore, it could not be regarded that the oral administration of cyclosporin is effective for treatment of psoriasis.

Another method for administration of cyclosporin for treatment of psoriasis is a subcutaneous injection. According to this method, when the preparation having cyclosporin concentration of 1.7mg/ml is subcutaneously injected thrice a week in an amount of 2ml, for 4 weeks, psoriasis can be effectively treated. However, this method has some disadvantages that it causes pain from injection and side effects including slight edema and therefore, it is very difficult to adapt the subject patient to the injection [see, V. C. Ho, C. E. M. Griffiths, C. N. Ellis, A. K. Gupta, C. C. McCuaig, B. J. Nickoloff, K. D. Cooper, T. A. Hamilton and J. J. Voorhees: Intralesional cyclosporin in the treatment of psoriasis, J. Am. Acad. Dermatol., 22(1), 94-100 (1990)].

Furthermore, it has been known that when cyclosporin is orally administered for a long time, severe side effects may be caused. Therefore, when the disease is limited to the skin, it is preferable that cyclosporin is administered topically, rather than systemically, to act directly on the target lesion, thereby the dose of cyclosporin to be administered can be reduced, the topical concentration of cyclosporin is increased to improve the pharmacological efficacy and further systemic side effects can be diminished.

However, contrary to the water-slouble or low molecular drugs it is very difficult to formulate cyclosporin into a topical preparation. The reason is that cyclosporin is a high molecular peptide compound having molecular weight more than 1200 and therefore, is difficult to penetrate into the lesion through the corneum. Due to such characteristic property of cyclosporin, it has been reported that most of the prior cyclosporin topical preparations cannot permeate the corneum for lack of skin permeability and therefore, cannot sufficiently provide the desired clinical effect [see, R. C. Hermann, R. S. Taylor, C. N. Ellis, N. A. Williams, N. D. Weiner, G. L. Flynn, T. M. Annesley and J. J. Voorhees: Topical cyclosporin for psoriasis; In vitro skin penetration and clinical study, Skin Pharmacol., 1, 246-249 (1988), J. I. Duncan, R. A. Wakeel, A. J. Winfield, A. D. Ormerod and A. W. Thomson: Immunomodulation of psoriasis with a topical cyclosporin A formulation, Acta. Derm. Venereol. (Stockh), 73(2), 84-87 (1993), Heule F., Laijendecker R. and van Joost : Topical cyclosporin A treatment in psoriasis and other dermatological diseases; theoretical and practical aspects. J. Dermatol. Treat., 2, 149-153 (1992)].

Therefore, it is very difficult to conclude that all of the cyclosporin topical preparations described in numerous prior references and patents exhibit a significant therapeutic effect on dermatologic diseases as mentioned above. The reason is that although it may be said that cyclosporin is effective for dermatologic diseases, any pharmacological effect cannot be determined unless the topical preparation permeates the corneum to reach the target lesion.

Japanese Laid-open Patent Publication No. (Hei) 5-310591 (November 22, 1993) discloses the cyclosporin topical preparation formulated by using a volatile lower alcohol. However, this preparation has disadvantages that the stability of the preparation during long-term storage is low and, when the preparation is applied to the skin, alcohol is rapidly volatilized to cause the precipitation of cyclosporin which results in the change of pharmacological efficacy.

WO 9520379 discloses the cyclosporin topical preparation using liposome. However, this preparation is substantially impossible to practically utilize since liposome is prepared using the method which is very difficult to apply to the field of industry.

Further, British Patent No. 2218334B teaches the cyclosporin topical preparation containing C₁₂₋₂₄ mono- or poly-fatty acid or alcohol as an absorption-enhancing agent. This preparation does not have sufficient skin permeability to the desired extent.

That is, the cyclosporin topical preparations proposed in the prior patents as mentioned above cannot attain the purpose of effectively utilizing cyclosporin in the form of a topical preparation and further are not systemically designed for the purpose of improving the skin permeability of cyclosporin.

Thus, the present inventors have extensively studied to develop a topical preparation which has an optimum skin permeability so that cyclospoin can efficiently reach the target lesion, i. e. derma portion, through corneum, and is pharmacologically useful and stable. Through many experiments we have identified that a cyclosporin topical preparation consisting of a certain composition as mentioned below shows a good skin permeability and stability and can attain the above mentioned purpose, and thus completed the present invention.

Therefore, it is an object of the present invention to provide a topical pharmaceutical composition containing cyclosporin wherein a self-emulsifying emulsion containing cyclosporin is dispersed in a hydrogel.

It is a further object of the present invention to provide a topical pharmaceutical composition containing cyclosporin, characterized in that an amphiphilic non-alcoholic solvent having high boiling point of 200°C or more is contained as a solubilizing and absorption-enhancing agent.

It is another object of the present invention to provide an emulsion-containing hydrogel preparation prepared by a process which comprises mixing a self-emulsifying emulsion preconcentrate (A) consisting of cyclosporin as an active ingredient, a solubilizing and absorption-enhancing agent selected from amphiphilic non-alcoholic solvents having high boiling point of 200°C or more, a surfactant and an oil, and a hydrogel (B) consisting of a gelling agent and purified water, and adjusting the pH value of the mixture with an alkalizing agent.

It is still another object of the present invention to provide a process for preparing the cyclosporin-containing topical pharmaceutical composition as defined above.

The foregoing has outlined some of the more pertinent objects of the present invention. These objects should be construed to be merely illustrative of some of the more pertinent features and applications of the invention. Many other beneficial results can be obtained by applying the disclosed invention in a different manner or modifying the invention within the scope of the disclosure. Accordingly, other objects and a more thorough understanding of the invention may be had by referring to the disclosure of invention and the drawings, in addition to the scope of the invention defined by the claims.

### BRIEF DESCRIPTION OF DRAWINGS

For a thorough understanding of the nature and objects of the invention, reference should be had to the following detailed description taken in connection with the accompanying drawings in which:
Figure 1 is a graph showing the transdermal permeability of the cyclosporin topical preparations depending on the kind of oils [ ● preparation of Example 5, ○ preparation of Example 8, ■ preparation of Example 1, □ preparation of Example 6, △ preparation of Example 7];
Figure 2 is a graph showing the transdermal permeability of the cyclosporin topical preparation depending on the kind of solubilizing and absorption-enhancing agents [ ● preparation of Example 2, ○ preparation of Example 1, ■ preparation of Comparative Example 2, □ Control preparation A];
Figure 3 is a graph showing the transdermal permeability of the cyclosporin topical preparation depending on the content of water [ ● preparation of Example 8 (85% water), ○ preparation of Example 9 (75% water), ■ preparation of Example 10 (90% water), □ preparation of Comparative Example 1 (0% water)]; and
Figure 4 is a graph showing the result of in vivo transdermal permeation test for the cyclosporin topical preparation using hairless mouse [ ■ preparation of Example 2 (cyclosporin 1%), □ control preparation (cyclosporin 5%)].

### DISCLOSURE OF INVENTION

In one aspect, the present invention relates to a topical pharmaceutical composition containing cyclosporin wherein a self-emulsifying emulsion containing cyclosporin is dispersed in a hydrogel.

Particularly, the present invention relates to a topical pharmaceutical composition containing cyclosporin, characterized in that an amphiphilic non-alcoholic solvent having high boiling point of 200°C or more is contained as a solubilizing and absorption-enhancing agent.

More particularly, the present invention relates to an emulsion-containing hydrogel preparation prepared by a process which comprises mixing a self-emulsifying emulsion preconcentrate (A) consisting of cyclosporin as an active ingredient, a solubilizing and absorption-enhancing agent selected from amphiphilic non-alcoholic solvents having high boiling point of 200°C or more, a surfactant and an oil, and a hydrogel (B) consisting of a gelling agent and purified water, and adjusting the pH value of the mixture with an alkalizing agent.

Hereinafter, each constituent of the topical pharmaceutical preparation according to the present invention will be more specifically explained.

### (i) Cyclosporin

The pharmacologically active ingredient used in the composition according to the present invention is cyclosporin. Although cyclosporin A, B, C, D, G and the like can be used as the cyclosporin component in the present invention, cyclosporin A is most preferable since its clinical effectiveness and pharmacological properties are well established in the art.

### (ii) Solubilizing and absorption-enhancing agent

The second essential component of the composition of the present invention is a solubilizing and absorption-enhancing agent. In the present invention, any of the amphiphilic (having both hydrophilic and lipophilic properties) non-alcoholic solvent having boiling point of 200°C or more can be suitably used as the solubilizing and absorption-enhancing agent, if they can increase the transdermal permeability of cyclosporin. Specifically, as the solubilizing and absorption-enhancing agent used in the present invention, a solvent satisfying the following requirements can be preferably selected: It has a suitable solubilizing effect on cyclosporin and acts as a cosurfactant owing to the fact that it has both hydrophilic and lipophilic properties, to help the emulsification of the preparation. In addition, it has a high boiling point, contrary to the lower alcohol having high volatility, so that its content is consistently retained during the storage period and therefore, the uniformity of the composition can be assured. Further, it is not volatilized even when it is applied to the skin to maintain the uniform composition, and increases the transdermal permeability of cyclosporin.

As the amphiphilic, i. e. both hydrophilic and lipophilic, non-alcoholic solvent having high boiling point of 200°C or more which can be used as the solubilizing and absorption-enhancing agent suitable to the skin permeation of cyclosporin in the present invention, it is identified that dimethylisosorbide (b.p. 234°C), propylene carbonate (b.p. 242°C) and N-alkyl-2-pyrrolidone wherein alkyl is C₁₋₆ alkyl, such as N-methyl-2-pyrrolidone (b.p. 202°C) are preferable.

Among these solubilizing and absorption-enhancing agents, N-methyl-2-pyrrolidone and dimethylisosorbide are most preferable.

In addition, for the purpose of controlling the transdermal permeability a combination of two or more solublizing and absorption-enhancing agents can also be used.

### (iii) Surfactant

As the surfactant in the composition of the present invention, various surfactants including pharmaceutically acceptable anionic, cationic, non-ionic or amphoteric surfactants which can stably emulsify the lipophilic components including cyclosporin, the oil component and the solubilizing and absorption-enhancing agent in a hydrogel to form a uniform emulsion can be used. Among these various surfactants, particularly, a non-ionic surfactant is preferable in view of its low skin irritability.

Particularly preferable surfactant which can be used in the present invention is a trans-esterification product of natural vegetable oil triglyceride and polyalkylene polyol, polyoxyethylene glycolated natural or hydrogenated vegetable oils, polyoxyethylene-sorbitan fatty acid esters, sorbitan fatty acid esters, polyoxyethylene alkyl ethers, etc. Typical example thereof is an esterification product of natural vegetable oil and polyoxyethylene glycol under trade name Labrafil (Gattefosse), a reaction product of castor oil and ethylene oxide under trade name Cremophor (BASF), polyoxyethylene-sorbitan mono- and tri-lauryl, palmityl, stearyl and oleyl esters under trade name Tween (ICI), sorbitan fatty acid ester under trade name Span (ICI) and polyoxyethylene alkyl ether under trade name Brij (BASF). Most preferable surfactant is mainly Labrafil M 1944 CS (apricot oil PEG-6 ester). In the composition of the present invention, although any one of the above-mentioned surfactants can be used alone, a combination of two or more surfactants can be preferably used to improve the emulsification state. For instance, a combination of Labrafil M 1944 CS and Cremophor EL (polyoxyethylene-35-castor oil) can be preferably used.

### (iv) Oil

As the oil component which can be used in the present invention, any of the oils of which the required HLB value in the oil-in-water(o/w) type emulsion is below 10 can be used. For example, medium chain fatty acid triglyceride (required HLB : about 5) such as caprylic/capric triglyceride, or natural vegetable oil (required HLB : 6-8) such as corn oil, coconut oil, cotton seed oil, soybean oil, rapeseed oil, safflower oil, sunflower oil, etc. can be used. In addition, refined fish oil as an animal oil can also be used. Further, for the purpose of improving the solubility of cyclosporin any additional surfactant having the required HLB of 10 or more such as castor oil (required HLB : about 14) or hard liquid paraffin (required HLB : 10-11) can also be combined.

### (v) Gelling agent

As the gelling agent in the composition of the present invention, any additive which can form a gel upon contact with water can be used. For example, cellulose derivative such as hydroxypropylmethyl cellulose (HPMC), hydroxypropyl cellulose (HPC), sodium carboxymethyl cellulose (Na·CMC), etc., carboxy vinyl polymer (trade name : Carbopol®, BF Goodrich Chemical), polyoxyethylene-polyoxypropylene copolymer (trade name : Pluronic®, BASF), etc. can be preferably used. Among them, carboxy vinyl polymer is particularly preferable.

### (vi) Purified water

The purified water which can be used in the present invention is a pharmaceutically acceptable graded water.

### (vii) Alkalizing agent

In the composition of the present invention, an alkalizing agent can be added to adjust the pH value, preferably to pH 3 to 9. For this purpose, any of inorganic alkaline substances, for example, Na₂HPO₄, NaHCO₃, Na₂CO₃, NaOH, etc., organic alkaline substances, for example, basic amino acids and amines can be used.

In the composition according to the present invention, each constituent is present in the ratio of (i) cyclosporin 0.1 to 10%, (i) solubilizing and absorption-enhancing agent 0.5 to 50%, (iii) surfactant 0.4 to 40%, (iv) oil 0.4 to 40%, (v) gelling agent 0.1 to 5%, (vi) purified water 0.1 to 90% and (vii) alkalizing agent 0.01 to 3.5%, preferaly in the ratio of (i) cyclosporin 0.1 to 5%, (ii) solubilizing and absorption-enhancing agent 0.5 to 25%, (iii) surfactant 0.4 to 20%, (iv) oil 0.4 to 20%, (v) gelling agent 0.1 to 2.5%, (vi) purified water 40 to 90% and (vii) alkalizing agent 0.05 to 1.8%, on the basis of a total weight of the composition. The composition of the present invention containing each constituent in the ratio as mentioned above is a topical preparation in the form of a hydrogel containing emulsion having the pH value of 3 to 9.

The topical pharmaceutical preparation containing cyclosporin according to the present invention can be prepared as follows:

First, cyclosporin (i) is dissolved in the solubilizing and absorption-enhancing agent (ii) and then mixed uniformly with the surfactant (iii) and the oil (iv) to prepare the self-emulsifying emulsion preconcentrate (A). Separately, the gelling agent (v) is dissolved in purified water (vi) with uniformly stirring to prepare the hydrogel (B). Then, the emulsion preconcentrate (A) is added portionwise to the hydrogel (B) with uniformly stirring to prepare the emulsion which is then mixed with the alkalizing agent (vii) to prepare the desired cyclosporin-containing topical pharmaceutical composition in the form of hydrogel containing emulsion.

The cyclosporin-containing topical pharmaceutical composition prepared according to the present invention has the following advantages:

First, the composition of the present invention has good stability and skin permeability. Since the cyclosporin topical preparation disclosed in the prior art contains a volatile lower alcohol (ethanol, isopropanol, etc.) as solvent [see, Japanese Laid-open Patent Publication No. (Hei) 5-310591 (November 22, 1993)], due to the volatility of solvent alcohol the stability of the preparation during long-term storage is low and further cyclosporin may be precipitated from the solution so that it cannot be transdermally absorbed to result in the change of the pharmacological efficacy of the preparation. Contrary to this, the present invention solves the problems involved in the prior preparations by utilizing a certain solubilizing and absorption-enhancing agent which (1) has a high boiling point, (2) has a good solubilizing effect for cyclosporin and (3) considerably increases the skin permeability of cyclosporin, to prepare the topical pharmaceutical composition which has a low volatility and a good stability and can continuously increase the skin permeability of the active ingredient, cyclosporin.

Second, the feeling when the composition of the present invention is applied to the skin is satisfactory. Since the composition of the present invention is a hydrogel preparation containing emulsion, it can be well spread on the skin. In addition, due to the moisturing effect of the solubilizing and absorption-enhancing agent itself, it is agreeable to the touch and has a well adaptability for the subject patient when it is applied to the skin.

Third, cyclosporin is very uniformly dispersed in the preparation. In general, it has been known that it is very difficult to uniformly disperse the emulsion particles having a diameter of 1 to 3µm. However, since the composition of the present invention is desigend in the form of a self-emulsifying hydrogel preparation, the emulsion particles can be readily and very uniformly dispersed in the viscous hydrogel base without any special control of temperature. Therefore, the present invention provides the preparation which is very stable for a long period and has a good skin permeability.

Fourth, the process for preparing the topical composition is very simple and convenient. In addition to the above-mentioned pharmaceutical advantages, since the composition of the present invention can be spontaneously emulsified to form an emulsion upon contact with water, the process of the present invention has advantages that the composition can be readily emulsified by simply stirring without any restriction of manufacturing condition such as temperature to uniformly disperse the emulsion particles in the hydrogel base and therefore, the uniform preparation can be constantly prepared.

The present invention will be more specifically illustrated by the following examples. However, it should be understood that the present invention is not limited by these examples in any manner.

### Example 1

| | |
|---|---|
| (A) Emulsion preconcentrate | |
| Cyclosporin | 10mg |
| Dimethylisosorbide | 50mg |
| Labrafil M 1944 CS | 37.5mg |
| Caprlic/capric triglyceride | 34.25mg |
| (B) Hydorgel | |
| Carbopol 940 | 13.5mg |
| Purified warer | 854.1mg |
| (C) Sodium hydroxide | 0.675mg |

The emulsion preconcentrate (A) was mixed with the hydrogel (B) with stirring and then aqueous sodium hydroxide solution (C) was added to prepare the gel preparation.

### Example 2

The topical pharmaceutical preparation was prepared using the following components according to the substantially same procedure as Example 1.

| | |
|---|---|
| (A) Emulsion preconcentrate | |
| Cyclosporin | 10mg |
| N-methyl-2-pyrrolidone | 50mg |
| Labrafil M 1944 CS | 37.5mg |
| Caprylic/capric triglyceride | 34.25mg |
| (B) Hydrogel | |
| Carbopol 940 | 13.5mg |
| Purified water | 854.1mg |
| (C) Sodium hydroxide | 0.675mg |

### Example 3

The topical pharmaceutical preparation was prepared using the following components according to the substantially same procedure as Example 1.

| | |
|---|---|
| (A) Emulsion preconcentrate | |
| Cyclosporin | 10mg |
| Dimethylisosorbide | 50mg |
| Labrafil M 1944 CS | 37.5mg |
| Corn oil | 34.25mg |
| (B) Hydrogel | |
| Carbopol 934 | 0.3mg |
| Purified water | 854.1mg |
| (C) Sodium hydroxide | 0.675mg |

### Example 4

The topical pharmaceutical preparation was prepared using the following components according to the substantially same procedure as Example 1.

| | |
|---|---|
| (A) Emulsion preconcentrate | |
| Cyclosporin | 50mg |
| N-methyl-2-pyrrolidone | 200mg |
| Labrafil M 1944 CS | 100mg |
| Cotton seed oil | 100mg |
| (B) Hydrogel | |
| Carbopol 940 | 20.0mg |
| Purified water | 528.5mg |
| (C) Sodium hydroxide | 1.5mg |

### Example 5

The topical pharmaceutical preparation was prepared using the following components according to the substantially same procedure as Example 1.

| | |
|---|---|
| (A) Emulsion preconcentrate | |
| Cyclosporin | 10mg |
| Dimethylisosorbide | 50mg |
| Labrafil M 1944 CS | 37.5mg |
| Refined fish oil | 34.25mg |
| (B) Hydrogel | |
| Carbopol 934 | 13.5mg |
| Purified water | 843.4mg |
| (C) Sodium carbonate | 11.4mg |

### Example 6

The topical pharmaceutical preparation was prepared using the following components according to the substantially same procedure as Example 1.

| | |
|---|---|
| (A) Emulsion preconcentrate | |
| Cyclosporin | 10mg |
| Dimethylisosorbide | 50mg |
| Labrafil M 1944 CS | 37.5mg |
| Castor oil | 34.25mg |
| (B) Hydrogel | |
| Carbopol 940 | 13.5mg |
| Purified water | 843.4mg |
| (C) Sodium carbonate | 11.4mg |

### Example 7

The topical pharmaceutical preparation was prepared using the following components according to the substantially same procedure as Example 1.

| | |
|---|---|
| (A) Emulsion preconcentrate | |
| Cyclosporin | 10mg |
| Dimethylisosorbide | 50mg |
| Labrafil M 1944 CS | 50mg |
| Hard liquid paraffin | 20mg |
| (B) Hydrogel | |
| Carbopol 940 | 13.5mg |
| Purified water | 845.1mg |
| (C) Sodium carbonate | 11.4mg |

### Example 8

The topical pharmaceutical preparation was prepared using the following components according to the substantially same procedure as Example 1.

| | |
|---|---|
| (A) Emulsion preconcentrate | |
| Cyclosporin | 10mg |
| Dimethylisosorbide | 50mg |
| Labrafil M 1944 CS | 37.5mg |
| Corn oil | 34.25mg |
| (B) Hydrogel | |
| Carbopol 940 | 13.5mg |
| Purified water | 843.4mg |
| (C) Sodium carbonate | 11.4mg |

### Example 9

The topical pharmaceutical preparation was prepared using the following components according to the substantially same procedure as Example 1.

| | |
|---|---|
| (A) Emulsion preconcentrate | |
| Cyclosporin | 10mg |
| Dimethylisosorbide | 50mg |
| Labrafil M 1944 CS | 37.5mg |
| Corn oil | 34.25mg |
| (B) Hydrogel | |
| Carbopol 940 | 13.5mg |
| Purified water | 470.0mg |
| (C) Sodium carbonate | 11.4mg |

### Example 10

The topical pharmaceutical preparation was prepared using the following components according to the substantially same procedure as Example 1.

| | |
|---|---|
| (A) Emulsion preconcentrate | |
| Cyclosporin | 10mg |
| Dimethylisosorbide | 50mg |
| Labrafil M 1944 CS | 37.5mg |
| Corn oil | 34.25mg |
| (B) Hydrogel | |
| Carbopol 940 | 13.5mg |
| Purified water | 1410mg |
| (C) Sodium carbonate | 11.4mg |

### Example 11

The topical pharmaceutical preparation was prepared using the following components according to the substantially same procedure as Example 1.

| | |
|---|---|
| (A) Emulsion preconcentrate | |
| Cyclosporin | 10mg |
| N-methyl-2-pyrrolidone | 100mg |
| Labrafil M 1944 CS | 50mg |
| Caprylic/capric triglyceride | 40mg |
| Cremophor EL | 30mg |
| (B) Hydrogel | |
| Carbopol 940 | 10mg |
| Purified water | 759.5mg |
| (C) Sodium hydroxide | 0.5mg |

### Example 12

The topical pharmaceutical preparation was prepared using the following components according to the substantially same procedure as Example 1.

| | |
|---|---|
| (A) Emulsion preconcentrate | |
| Cyclosporin | 10mg |
| N-methyl-2-pyrrolidone | 100mg |
| Labrafil M 1944 CS | 40mg |
| Castor oil | 50mg |
| Cremophor EL | 40mg |
| (B) Hydrogel | |
| Carbopol 940 | 10mg |
| Purified water | 749.5mg |
| (C) Sodium hydroxide | 0.5mg |

### Example 13

The topical pharmaceutical preparation was prepared using the following components according to the substantially same procedure as Example 1.

| | |
|---|---|
| (A) Emulsion preconcentrate | |
| Cyclosporin | 30mg |
| N-methyl-2-pyrrolidone | 150mg |
| Labrafil M 1944 CS | 105mg |
| Caprylic/capric triglyceride | 90mg |
| Cremophor EL | 30mg |
| Castor oil | 30mg |
| (B) Hydrogel | |
| Carbopol 940 | 10mg |
| Purified water | 554.5mg |
| (C) Sodium hydroxide | 0.5mg |

### Example 14

The topical pharmaceutical preparation was prepared using the following components according to the substantially same procedure as Example 1.

| | |
|---|---|
| (A) Emulsion preconcentrate | |
| Cyclosporin | 10mg |
| N-methyl-2-pyrrolidone | 50mg |
| Propylene carbonate | 10mg |
| Labrafil M 1944 CS | 35mg |
| Caprylic/capric triglyceride | 30mg |
| Cremophor EL | 10mg |
| Castor oil | 10mg |
| (B) Hydrogel | |
| Carbopol 940 | 10mg |
| Purified water | 834.5mg |
| (C) Sodium hydroxide | 0.5mg |

### Comparative Example 1

The topical pharmaceutical preparation was prepared using the following components according to the substantially same procedure as Example 1.

| | |
|---|---|
| (A) Emulsion preconcentrate | |
| Cyclosporin | 10.0mg |
| Dimethylisosorbide | 406.6mg |
| Labrafil M 1944 CS | 304.9mg |
| Corn oil | 278.5mg |

### Comparative Example 2

The topical pharmaceutical preparation was prepared using the following components according to the substantially same procedure as Example 1.

| | |
|---|---|
| (A) Emulsion preconcentrate | |
| Cyclosporin | 10mg |
| Labrafil M 1944 CS | 62mg |
| Corn oil | 56mg |
| (B) Hydrogel | |
| Carbopol 940 | 13.5mg |
| Purified water | 847.1mg |
| (C) Sodium carbonate | 11.4mg |

### Experiment 1

### In vitro transdermal permeation test using hairless mouse

To determine the skin permeability of the cyclosporin-containing topical preparation according to the present invention the concentration of cyclosporin in the receiving solution was measured at scheduled time intervals using hairless mouse in Franz cell. Specific experimetal procedure is described in the following.

Hairless mouse (5 weeks aged male mouse) was anesthesized with subcutaneous injection of 20% urethane solution in an amount of 1.2g/kg and then the abdominal skin was cut down and used as the membrane in Franz cell (surface area : 3.14cm², volume of receiver : 13ml). 40% Propylene glycol solution was used as the receiving solution. The topical preparation to be tested was spread on the skin of hairless mouse in an amount corresponding to 500µg of cyclosporin and then under condition of 600rpm 300µl of the receiving solution was taken at scheduled time intervals with 300µl of fresh receiving solution being supplemented each time. The separated receiving solution was analyzed with HPLC under the following condition:
Column : µ-Bondapak® C18 (3.9X300mm, Waters)
Detector : UV 210nm
Mobile phase : acetonitrile:methanol:water = 70:15:35
Flow rate : 1.2ml/min.
Column temperature : 70°C
Injection volume : 100µl

### 1. Transdermal permeation test depending on the kind of oil:

The effect of oil on the transdermal permeability was determined by measuring the transdermal permeability of the cyclosporin-containing topical preparation with varying the kind of oils according to the above-mentioned method. In this test, the topical preparations of Examples 1, 5, 6, 7 and 8 were used as the test preparation.

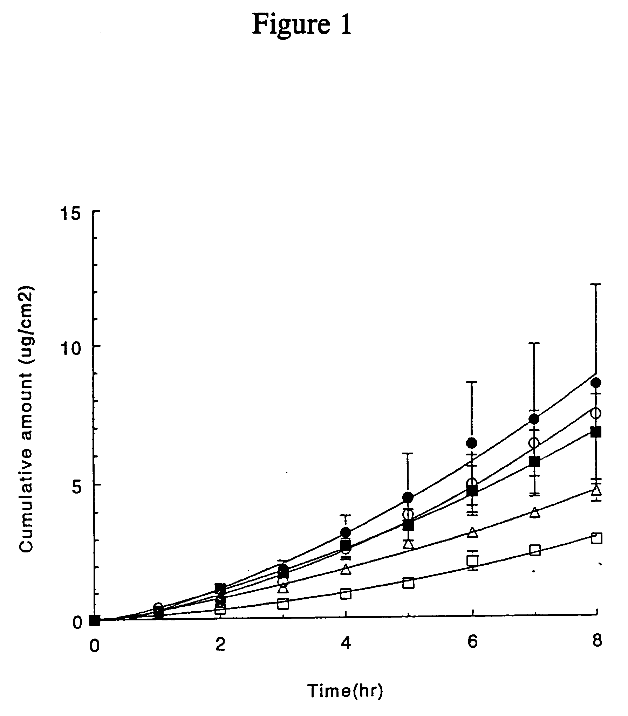

The result as measured is depicted in Figure 1. As can be seen from the result depicted in Figure 1, in the transdermal permeation test using refined fish oil (Example 5), corn oil (Example 8), castor oil (Example 6), hard liquid paraffin (Example 7) and medium chain fatty acid triglyceride (Example 1) as the oil component, the oils having low required HLB value in oil-in-water(o/w) type emulsion, for example, refined fish oil, corn oil and medium chain fatty acid triglyceride show better skin permeability than castor oil and hard liquid paraffin.

### 2. Transdermal permeation test depending on the kind of solubilizing and absorption-enhancing agent:

The effect of solubilizing and absorption-enhancing agent on the transdermal permeability was determined by measuring the transdermal permeability of the cyclosporin-containing topical preparation with varying the kind of solubilizing and absorption-enhancing agents according to the above-mentioned method. In this test, the topical preparations of Examples 1, 2, 11 and 12 were used as the test preparation. As the control preparation, the preparation of Comparative Example 2 and the control preparation A having the following composition as prepared according to Example 1.1 of British Patent No. 2218334B were used:

### Control preparation A:

| Component | Content (wt%) |
|---|---|
| Cyclosporin | 20 |
| Vaccenyl alcohol | 20 |
| Isopropyl alcohol | 40 |
| Tween 80 | 20 |
| Aerosil (SiO₂) | q.s. |

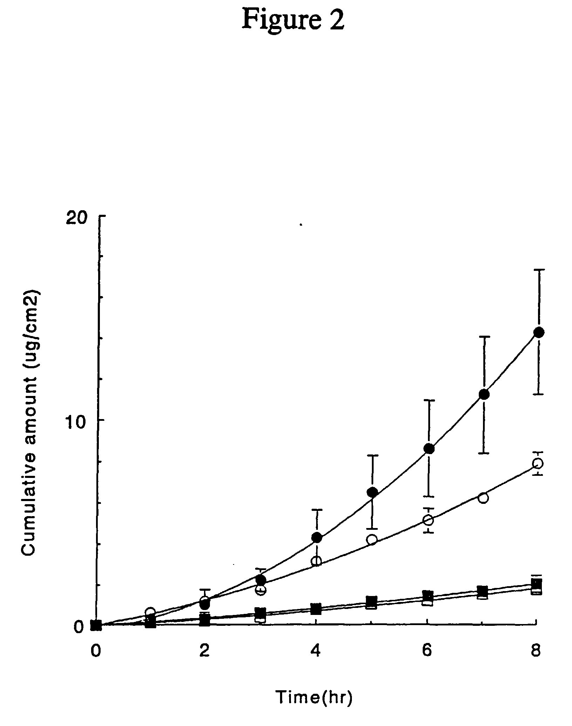

The result as measured is depicted in Figure 2. As can be seen from the result depicted in Figure 2, the present preparation using the solubilizing and absorption-enhancing agent such as dimethylisosorbide (Example 1) and N-methyl-2-pyrrolidone (Example 2) shows considerably better skin permeability than the preparation of Comparative Example 2 and the control preparation A using C₁₂₋₂₄ fatty acid and fatty alcohol. Accordingly, it can be determined that the selection of the solubilizing and absorption-enhancing agent according to the present invention is essential to make the cyclosporin topical preparation.

### 3. Transdermal permeation test depending on the content of water:

The effect of the content of water on the transdermal permeability was determined by measuring the transdermal permeability of the cyclosporin-containing topical preparation with varying the content of water according to the above-mentioned method. In this test, the topical preparations of Examples 8, 9 and 10 were used as the test preparation and the preparation of Comparative Example 1 was used as the control preparation.

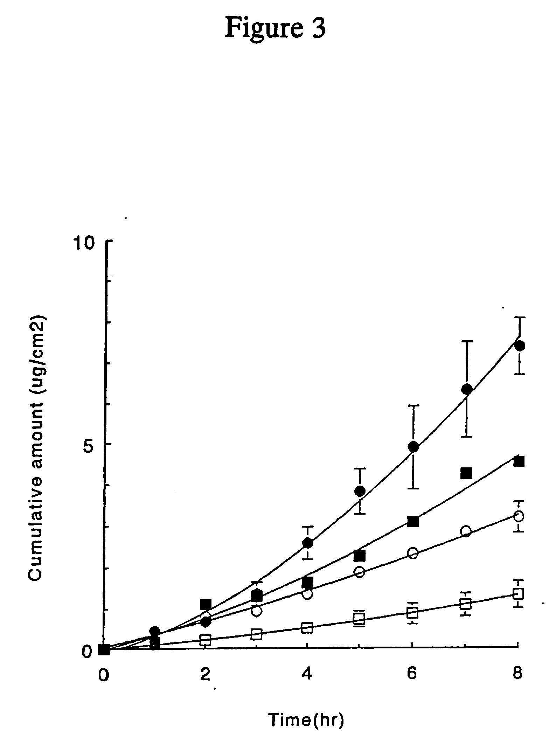

The result as measured is depicted in Figure 3. As can be seen from the result depicted in Figure 3, the preparation of Comparative Example 1 not containing water shows extremely low skin permeability. Accordingly, it can be considered that the skin permeation needs at least a certain degree of water which is necessary for the hydration of skin, and therefore, the composition of the present invention in which the emulsion is uniformly dispersed in the hydrogel containing water is suitable to the skin permeation.

### Experiment 2

### In vivo transdermal permeation test using hairless mouse

The skin permeation of the cyclosporin-containing topical preparation according to the present invention was compared in vivo with the 5% cyclosporin-containing preparation according to Japanese Laid-open patent Publication No. (Hei) 5-310591, which has been known as having good skin penetration and corneum permeability to exhibit the desired pharmacological activity, as the control preparation. In this test, the concentration of cyclosporin in viable skin tissue was measured using hairless mouse at scheduled time intervals. Specific experimental procedure is as follows. To prevent the leak of the preparation silicon (Shin-Etsu silicone, general RTV rubber, KE45W, deoxime type 100g, Korea) was spread on the abdominal skin of hairless mouse in dorsal position so that the spreading area of the preparation is 1cm²(1cmX1cm) and then each of the test preparation and the control preparation was uniformly spread thereon in an amount of about 0.04g/cm².

After 2, 8, 14 and 24 hours from application of the preparation, the skin was wiped with cotton dipped in ethanol twice and then with dry cotton. The skin was wiped once with cotton dipped in water and then the corneum was stripped off 20 times using scotch tape (3M Scotch 810 tape). The viable tissue was cut down as much as the area on which the preparation is spread and then the amount of the absorbed preparation was measured with HPLC according to the following analysis method.

The amount of the active component in the viable tissue was analyzed according to the following method. Specifically, the separated viable tissue was introduced into 10ml of 0.15M Tris buffer (pH 7.6) and homogenized at 10,000rpm for 10 minutes with Teflon pestle. The obtained homogenate was centrifuged at 10,000rpm for 5 minutes and 3ml of the supernatant was removed. To this supernatant was added 100µl of the internal standard solution (containing 20µg of cyclosporin D per ml) and then 2ml of diethylether. The mixture was agitated for 2 minutes and 1.5ml of the supernatant was removed and developed on activated Bakerbond silica gel column (J. T. Baker, U.S.A.). The column was washed with 3ml of n-hexane and the active component was eluted with 3ml of methanol. The eluant was dried under N₂ gas and 200µl of the mobile phase was added thereto. 100µl of the soilution was subjected to HPLC. The condition for HPLC are as follows:
Column : µ-Bondapak® C18 (3.9X300mm, Waters)
Detector : UV 210nm
Mobile phase : acetonitrile:methanol:water=58:15:40
Flow rate : 1.0ml/min.
Column temperature : 70°C
Injection volume : 100µl

In this test, the topical preparation prepared in Example 2 (preparation containing 1% cyclosporin) was used as the test preparation and the topical preparation having the following composition according to Example 6(3) of Japanese Laid-open Patent Publication No. (Hei) 5-310591 (November 22, 1993) was used as the control preparation:

| Component | Content (wt%) |
|---|---|
| Cyclosporin | 5 |
| 95% Ethanol | 2 |
| Isopropyl myristate | 5 |
| Olive oil | 48 |
| Polyoxyethylene glycol (5) monostearate | 35 |
| Aerosil | 5 |

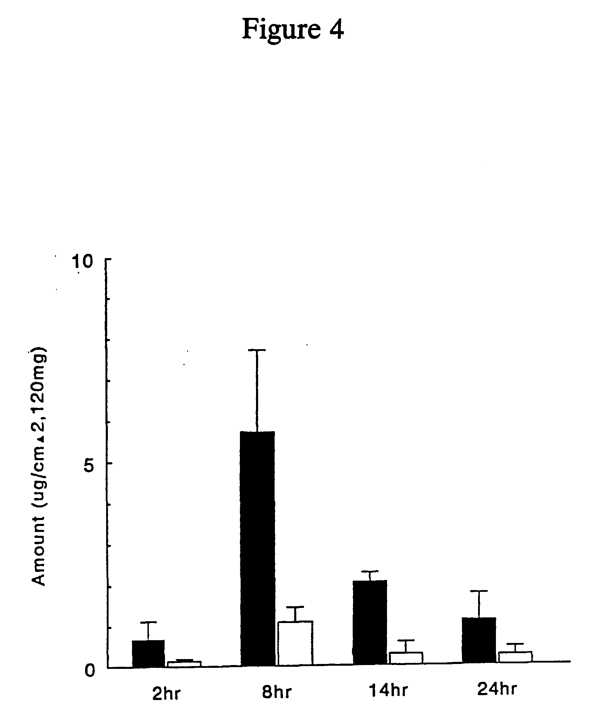

The result as measured is depicted in Figure 4. As can be seen from the result depicted in Figure 4, the concentration of cyclosporin in the viable tissue treated with the topical preparation (cyclosporin 1% preparation) according to the present invention is much higher than that with the control preparation (cyclosporin 5% preparation). Accordingly, it can be identified that the cyclosporin-containing topical preparation according to the present invention shows significantly superior transdermal permeability in comparison to the prior preparations.

Although this invention has been described in its preferred form with a certain degree of particularity, it is appreciated by those skilled in the art that the present disclosure of the preferred form has been made only by way of example and that numerous changes in the details of the construction, combination and arrangement of parts may be resorted to without departing from the spirit and scope of the invention.

## Claims

1. A topical pharmaceutical composition containing cyclosporin wherein a self-emulsifying emulsion containing cyclosporin is dispersed in a hydrogel.

2. The topical pharmaceutical composition containing cyclosporin as defined in claim 1, which comprises an amphiphilic non-alcoholic solvent having high boiling point of 200°C or more as a solubilizing and absorption-enhancing agent.

3. The topical pharmaceutical composition containing cyclosporin as defined in claim 2, wherein the cyclosporin is cyclosporin A.

4. The topical pharmaceutical composition containing cyclosporin as defined in claim 2, wherein the solubilizing and absorption-enhancing agent is selected from the group consisting of dimethylisosorbide, propylene carbonate and N-alkyl-2-pyrrolidone in which alkyl has 1 to 6 carbon atom.

5. The topical pharmaceutical composition containing cyclosporin as defined in claim 4, wherein a mixture of two or more solubilizing and absorption-enhancing agents is used.

6. The topical pharmaceutical composition containing cyclosporin as defined in claim 4, wherein the solubilizing and absorption-enhancing agent is dimethylisosorbide or N-methyl-2-pyrrolidone.

7. The topical pharmaceutical composition containing cyclosporin as defined in claim 6, wherein the solubilizing and absorption-enhancing agent is N-methyl-2-pyrrolidone.

8. The topical pharmaceutical composition containing cyclosporin as defined in claim 1 or 2, which further comprises one or more substances selected from the group consisting of an surfactant, an oil, a gelling agent, a purified water and an alkalizing agent.

9. The topical pharmaceutical composition containing cyclosporin as defined in claim 8, wherein the surfactant is selected from the group consisting of trans-esterification product of natural vegetable oil triglyceride and polyalkylene polyol, polyoxyethylene glycolated natural or hydrogenated vegetable oil, polyoxyethylene-sorbitan fatty acid ester, sorbitan fatty acid ester and polyoxyethylene alkyl ether.

10. The topical pharmaceutical composition containing cyclosporin as defined in claim 9, wherein the surfactant is a trans-esterification product of natural vegetable oil triglyceride and polyalkylene polyol.

11. The topical pharmaceutical composition containing cyclosporin as defined in claim 8, wherein the oil is a medium chain fatty acid triglyceride, a vegetable oil or an animal oil, which has the required HLB value up to 10 in oil-in-water(o/w) type emulsion.

12. The topical pharmaceutical composition containing cyclosporin as defined in claim 11, wherein the oil is refined fish oil, corn oil or caprylic/capric triglyceride.

13. The topical pharmaceutical composition containing cyclosporin as defined in claim 8, wherein the oil is a mixture of an oil having the required HLB value up to 10 in oil-in-water (o/w) type emulsion and an oil having the required HLB value more than 10 in oil-in-water (o/w) type emulsion.

14. The topical pharmaceutical composition containing cyclosporin as defined in claim 8, wherein the gelling agent is selected from the group consisting of cellulose derivative, carboxy vinyl polymer and polyoxyethylene-polyoxypropylene copolymer.

15. The topical pharmaceutical composition containing cyclosporin as defined in claim 14, wherein the gelling agent is carboxy vinyl polymer.

16. The topical pharmaceutcial composition containing cyclosporin as defined in claim 8, wherein cyclosporin 0.1 to 10%, the solubilizing and absorption-enhancing agent 0.5 to 50%, the surfactant 0.4 to 40%, the oil 0.4 to 40%, the gelling agent 0.1 to 5%, purified water 0.1 to 90% and the alkalizing agent 0.01 to 3.5% are combined on the basis of a total weight of the composition.

17. The topical pharmaceutical composition containing cyclosporin as defined in claim 16, wherein cyclosporin 0.1 to 5%, the solubilizing and absorption-enhancing agent 0.5 to 25%, the surfactant 0.4 to 20%, the oil 0.4 to 20%, the gelling agent 0.1 to 2.5%, purified water 40 to 90% and the alkalizing agent 0.05 to 1.8% are combined on the basis of a total weight of the composition.

18. The topical pharmaceutical composition containing cyclosporin as defined in claim 2, wherein pH value of the composition is within the range of 3 to 9.

19. A process for preparing the topical pharmaceutical composition containing cyclosporin as defined in any one of claims 1 to 18, which comprises dissolving cyclosporin in a solubilizing and absorption-enhancing agent, mixing the resulting solution with a surfactant and an oil to prepare a self-emulsifying emulsion preconcentrate, mixing the resulting emulsion preconcentrate with a hydrogel formed by uniformly dissolving a gelling agent in purified water and stirring the mixture, and then adding an alkalizing agent to the mixture to prepare a hydrogel preparation containing emulsion.
